# EUROPEAN PATENT SPECIFICATION

(11) **EP 0 490 459 B1**
(45) Date of publication and mention of the grant of the patent: **19.02.1997**
(21) Application number: 91203298.4
(22) Date of filing: 13.12.1991
(51) Int. Cl.: A61M 25/00

(54) **Multilumen catheter**
Mehrlumiger Katheter
Cathéter à lumières multiples

(30) Priority: 14.12.1990 US 627534
(43) Date of publication of application: 17.06.1992
(73) Proprietor: THE KENDALL COMPANY, Mansfield, Massachusetts 02048 (US)
(72) Inventor: Gross, James R., Wareham, Massachusetts 02571 (US)
(74) Representative: Smulders, Theodorus A.H.J., Ir.

(56) References cited:
- EP-A- 0 161 863
- EP-A- 0 244 955
- WO-A-88/10128
- DE-A- 3 112 762
- US-A- 4 705 501
- US-A- 4 737 145

## Description

The present invention relates to a multilumen catheter comprising an inner catheter tube defining a first lumen having a proximal end and a distal end; a middle catheter tube defining a second lumen having a proximal end, a distal end and a cross-section which is greater than the cross-section of said inner tube, said middle tube being disposed concentrically and coaxially with said inner tube, and said distal end of said inner tube extending longitudinally outward from said distal end of said middle tube; an outer catheter tube defining a third lumen and having a proximal end, a distal end and a cross-section which is greater than the cross-section of said middle tube, said outer tube being disposed concentrically and coaxially with said middle tube and said inner tube; a manifold securing and encapsulating each of said proximal ends of said middle and outer tubes; and a plurality of extension tubes coupled to said manifold, each separately coupled to the second and third lumen respectively.

Multilumen venous catheters are advantageous as they eliminate the need for separate catheters for monitoring a patient's blood pressure and infusing or withdrawing fluids from a patient thereby decreasing the possibility of patient discomfort and possible infection inherent with the insertion of several single lumen catheters into the patient. Maintaining fluids separately is particularly critical when the fluids employed are chemically incompatible or when cross contamination of fluids is to be avoided.

Such a multilumen catheter is disclosed in DE-A-3112762, which is used as a basis for the preamble of the independent claims and which shows a multilumen catheter comprising an inner, middle and outer catheter tube with respective first, second and third lumens. The tubes are disposed concentrically and coaxially with each other; a distal end of the inner tube extends longitudinally outward from a distal end of the middle tube; a distal end of the outer tube extends longitudinally from the distal end of the middle tube. A screw mechanism holds the inner and middle tube together at their proximal ends. To prevent the inner tube from slipping out, it is provided with an annular enlargement fixed by a screwed cap.

Annother catheter device, known from WO-A-8810128, includes a longitudinally extending end portion with a catheter tube distal terminus for insertion into a vein. A plurality of independent lumens extend through the catheter, preferably including a distal lumen, a middle lumen and a proximal lumen, each of the lumens having a wall, a proximal end, and a lumen distal terminus adjacent to the catheter tube distal terminus. Each lumen distal terminus is spaced longitudinally from each other so as to include a distal, a middle and a proximal lumen terminus, so that fluids can be infused simultaneously into the bloodstream without mixing prior to entry into the bloodstream.

These conventional multilumen catheters may be potentially hazardous to the patient as they provide a direct conduit for blood to leave the patient, and have been responsible for causing substantial blood loss. Some conventional multilumen catheters are equipped with extension tubing coupled to valves which close the separate lumens to blood flow when the lumens are not in use. However, these valves are not always reliable.

Another disadvantage of conventional multilumen catheters is that they are usually made in one piece from a material of limited flexibility and softness. Thus, the patient may experience increased trauma and discomfort from a relatively rigid catheter as the catheter is inserted in a vein, and damage to the inner lining of the vein may also result from such relatively rigid catheters.

Therefore, it would be desirable to have a multilumen catheter which overcomes the above-described disadvantages, which is economical to produce, and which is compatible with existing medical equipment used with conventional multiple lumen catheters.

To this effect according to a first aspect of the invention a multilumen catheter with the characteristic features of claim 1 is provided and according to a second aspect of the invention with the characteristic features of claim 2 is provided.

In accordance with the present invention, disclosed is a multilumen catheter having a plurality of fexible, elongated catheter tubes concentrically and coaxially disposed, each catheter tube defining a separate lumen, each catheter tube having a distal end which extends outwardly from the distal end of the next surrounding catheter tube, and each catheter tube having a proximal end which is secured and encapsulated by a manifold which facilitates fluid communication between each of the lumens and separates fluid transfer devices or pressure monitoring devices via extension tubing.

The concentric arrangement of the catheter tube provides an efficient cross-sectional area relative to the overall cross-sectional area. Furthermore, the smallest catheter tube whose distal end extends outwardly the furthest may be made from a softer, more flexible material so as to provide an overall softer distal end of the multilumen catheter.

In one embodiment of the present multilumen catheter, each of the plurality of catheter tubes has a distal end which terminates in a tapered flexible tip. The flexible tip remains closed when there is no fluid within the respective lumen thereby eliminating the possibility of substantial blood loss through the present multilumen catheter.

This invention will be more fully understood from the following solely exemplary detailed description taken in conjunction with the accompanying drawings in which:
Fig. 1A is a view, partly in section, of the multilumen catheter of the present invention showing the concentrically arranged catheter tubes, their distal terminal, and the manifold, wherein the tapered tips of the tubes however are not visible;
Fig. 1B is a cross-sectional view of the multilumen catheter of the present invention;
Fig. 2 is a perspective view of an embodiment of the distal end of the multilumen catheter of Fig. 1A;
Fig. 3 is a perspective view of another embodiment of the distal end of the multilumen catheter of Fig. 1A;
Fig. 4 is a perspective view of yet another embodiment of the distal tip of the multilumen catheter shown in Fig. 1A; and
Fig. 5 is a view, partly in section, showing the bottom half of an alternative embodiment of the manifold of the multilumen catheter shown in Fig. 1A.

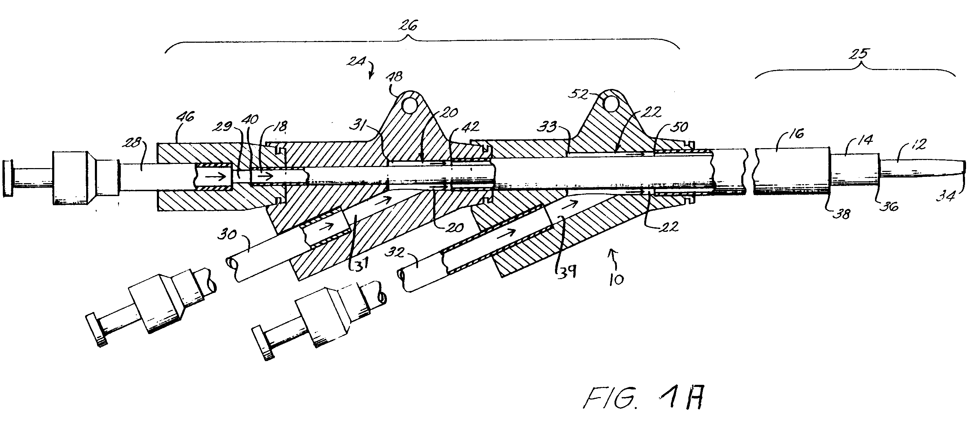

Referring now to Figs. 1A-1B, one embodiment of the multilumen catheter in accordance with the present invention is shown. The present multilumen catheter 10 comprises three concentrically and coaxially disposed, flexible, elongated catheter tubes 12, 14, 16, each defining separate lumens 18, 20, 22 as shown in cross-section in Fig. 1B.

The inner catheter tube 12 has a cross-section of about 1.168 mm (0.046 in.) (ID) and a wall thickness of 1.27 mm (0.05 in.); and extends longitudinally outward from the distal end 36 of the middle catheter tube 14. The middle catheter tube 14 has a cross-section of about 1.651 mm (0.065 in.) (ID), a wall thickness of 0.127 mm (0.005 in.), and extends longitudinally outward from the distal end 38 of the outer catheter tube 16. The outer tube 16 has a cross-section of about 20.828 mm (0.82 in.) (ID), and a wall thickness of 0.127 mm (0.005 in.) Accordingly, tube 12 is equivalent to a 16 gauge tube, and tubes 14 and 16 are equivalent to an 18 gauge tube.

It should be understood that more than three catheter tubes may be similarly arranged. However, for exemplary purposes, one embodiment of the present invention having three concentric lumens is shown and described in detail.

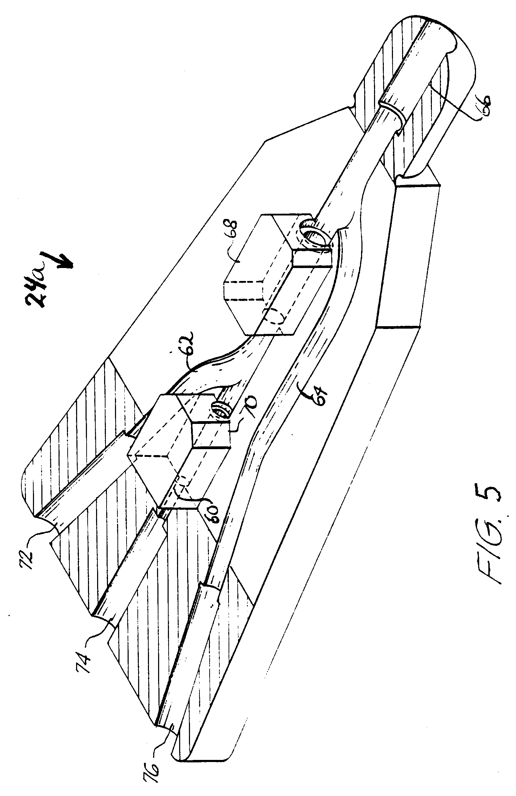

Separate lumens 18, 20, 22 terminate coextensively with the respective distal ends of the catheter tubes 12, 14, 16. Such a termination of the lumens 18, 20, 22, can be more clearly seen in Fig. 4, 5 which is a view of the distal end 25 of the multilumen catheter 10 shown in Fig. 1A.

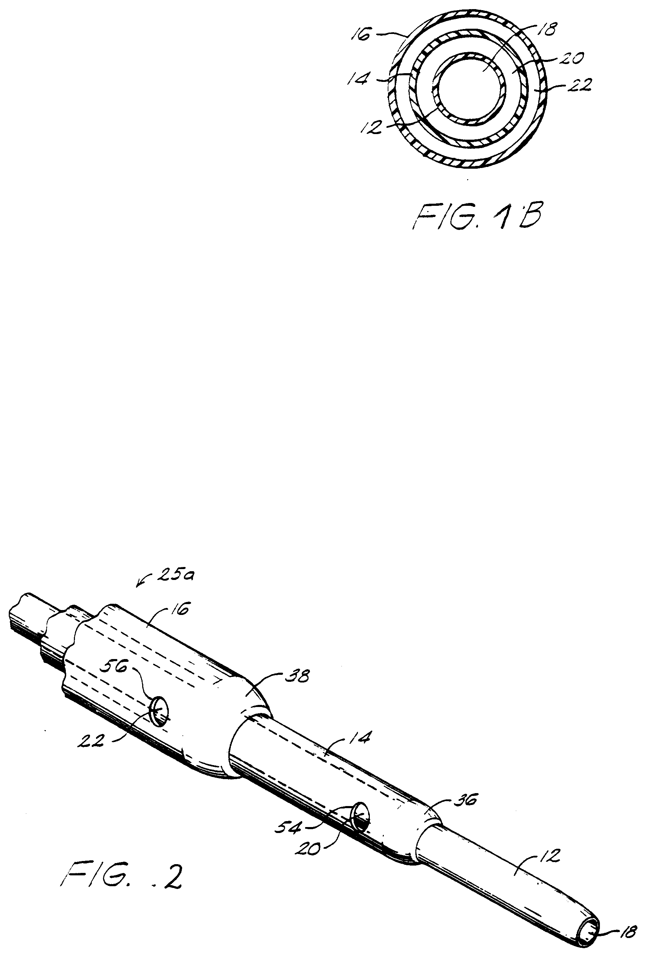

In accordance with the invention, lumens 18, 20, 22 are independent and noncommunicative with one another, and various fluids simultaneously carried thereby do not mix prior to entering the blood stream. Furthermore, as can be clearly seen in Fig. 2, the distal ends 34, 36, 38 of catheter tubes 12, 14, 16 are spaced apart from one another, which ensures that fluids simultaneously carried within each of the lumens 18, 20, 22 do not mix prior to being assimilated in the blood stream.

Suitable materials from which catheter tubes 12, 14, 16 may be made include flexible, sterilizable materials such as polyurethane, silicone, polyvinyl chloride (PVC) and nylon (registered trade mark). Polyurethane is preferred. Furthermore, the inner tube 12 and the middle tube 14 may be formed from a less rigid material such as a softer, more flexible polyurethane thereby providing an overall softer distal end 25 of the multilumen catheter 10.

A softer distal end 25 is particularly desirable as it reduces trauma to the sensitive inner lining of the vein into which a multilumen catheter 10 may be inserted. Furthermore, a soft, flexible distal end 25 facilitates insertion of the catheter 10 into sinuous veins which may otherwise be difficult to access using conventional catheters having more rigid distal ends.

Referring again to Fig. 1A, the proximal end 26 of the multilumen catheter 10 includes a manifold 24 which encapsulates and secures catheter tubes 12, 14, 16 and further provides fluid communication between the lumens 18, 20, 22 and fluid transfer devices or pressure monitoring devices via separate extension tubing 28, 30, 32. Extension tubing 28, 30, 32 may be configured for attachment to fluid transfer devices or pressure monitoring devices as is known in the art. Extension tubing may be made from any suitable medical grade tubing.

As shown in the illustrative drawing, the proximal end 40 of the inner tube 12 preferably extends beyond the proximal end 42 of the middle tube 14 and is encapsulated within a hub 46 in fluid communication with extension tubing 28 via a channel 29 between the proximal end 40 of inner tube 12 and extension tubing 28. Likewise, the proximal end 42 of the middle tube 14 preferably extends beyond the proximal end 50 of the outer tube 16 and is encapsulated within a hub 48 in fluid communication with extension tubing 30 via a channel 37 communicating with an annular space 31 between the proximal end 42 of the middle tube 14 and extension tubing 30. The proximal end 50 of the outer tube 16 is encapsulated within hub 52 in fluid communication with extension tubing 32 via a channel 39 communicating with an annular space 33 between the proximal end 50 of outer tube 16 and extension tubing 32.

Each of the hubs 46, 48, 52 may be formed from a rigid, sterilizable, plastic material which can be molded using known techniques. Such materials include polycarbonate, polyurethane and PVC. The hubs 46, 48, 52, may be separately formed and then sealed together by known means to form the manifold 24.

The path of fluids flowing through the manifold 24 is indicated by arrows in Fig. 1A. Fluids flow from extension tubing 28 into the channel 29 and thereafter flow through lumen 18. Fluids flow from extension tubing 30 into the channel 37 and annular space 31 and thereafter flow around the inner tube 12 and through lumen 20. Similarly, fluids flow from extension tubing 32 into the channel 39 and annular space 33 and thereafter flow around middle tube 14 and through lumen 22.

Referring now to Fig. 2, the distal end 25a of the multilumen catheter 10 according to Fig. 1A is shown. In this embodiment, the distal end 36 of the middle tube 14 is tapered and abuts the inner tube 12 which outwardly extends beyond the middle tube 14. The lumen 20 defined by the middle tube 14 terminates in a side opening 54 slightly upstream from the tapered distal end 36 of the middle tube 14.

Furthermore, the distal end 38 of the outer tube 16 is tapered and abuts the middle tube 14. The lumen 22 defined by the outer tube 16 terminates in a side opening 56 slightly upstream from the tapered distal end 38 of the outer tube 16.

In accordance with the present invention, the side openings 54, 56 enable lumens 20, 22 to aspirate fluids from the patient as well as deliver fluids to the patient. The lumen 18 defined by the inner tube 12 may serve to deliver fluids or to monitor blood pressure.

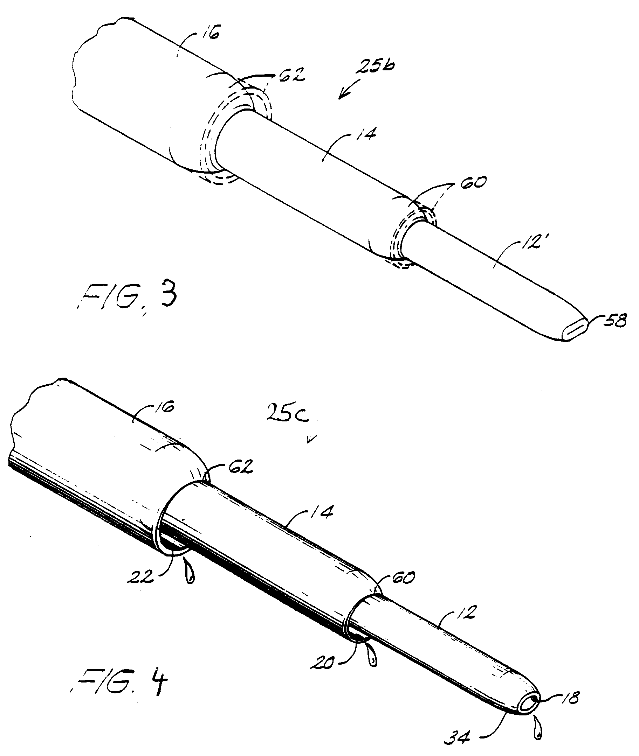

Referring now to Fig. 3, another embodiment of the distal end 25b of the multilumen catheter is shown. In this embodiment, inner tube 12, the middle tube 14 and the outer tube 16 each terminate in tapered flexible tips 58, 60, 62. The flexible tips 58, 60, 62, expand under pressure exerted from fluids flowing through the respective lumens to allow fluids to exit from the lumens into the bloodstream. However, when no fluids are present in any one or more of the lumens, the flexible tips associated with those lumens remain closed. When closed, the tips 58, 60, 62 act as check valves to prevent the backflow of blood through the lumens thereby completely eliminating the possibility of substantial blood loss through the present multilumen catheter.

Referring now to Fig. 4, yet another embodiment of the distal end 25c of the multilumen catheter is shown. This embodiment is similar to that shown in Fig. 3 except the inner catheter tube 12 does not terminate in a flexible tip and instead remains open. This enables the lumen 18 defined by the inner tube 12 to be used to monitor blood pressure which would not be possible if the distal end 34 of the inner catheter tube 12 were closed.

In accordance with the present invention, the different embodiments of the distal end of the present multilumen catheter tube as shown in Figs. 1A-4 may be combined as desired in a single multilumen catheter of the present invention. For example, the outer tube may have a distal end which terminates in a side opening as shown in Fig. 2 while the middle tube terminates in a flexible tip as shown in Fig. 4.

Referring now to Fig. 5, the bottom half of an alternative embodiment of the manifold 24a is shown. In this embodiment, manifold 24a is molded using known techniques. The manifold 24a secures the respective proximal ends of the catheter tubes. The proximal end of the outer tube is secured at the base 66 of the manifold 24a, the proximal end of the middle tube is secured at the center 68 of the manifold 24a and the proximal end of the inner tube is secured at the top 70 of the manifold 24a. Molded channels 60, 62, 64 separately communicate with the respective lumens defined by each of the catheter tubes. Extension tubes may be molded or otherwise secured into the bottom half of the manifold 24a at channels 72, 74, 76 which are respectively coupled to channels 60, 62, 64 within the manifold.

The inner tube, the middle tube, the outer tube and extension tubing are permanently affixed within the manifold using known techniques. A cooperative top half of the manifold is sealed to the bottom half to provide a sealed, liquid tight unit. Fluids entering or leaving the manifold via the extension tubing communicate with the respective lumens defined by each of the catheter tubes through molded channels 60, 62, 64.

It will be understood that a number of manufacturing techniques known in the art may be used to form the multilumen catheter of the present invention including the various embodiments described above.

The manner of using the multilumen catheter of the present invention is substantially the same as using a conventional multilumen catheter of the prior art, which is well known to those skilled in the art and need not be described in detail.

This invention is not to be limited by what has been particularly shown and described, except as indicated by the appended claims.

## Claims

1. Multilumen catheter (10) comprising an inner catheter tube (12) defining a first lumen (18) having a proximal end (40) and a distal end (34); a middle catheter tube (14) defining a second lumen (20) having a proximal end (42), a distal end (36) and a cross-section which is greater than the cross-section of said inner tube (12), said middle tube (14) being disposed concentrically and coaxially with said inner tube (12), and said distal end (34) of said inner tube (12) extending longitudinally outward from said distal end (36) of said middle tube (14); an outer catheter tube (16) defining a third lumen (22) and having a proximal end (50), a distal end (38) and a cross-section which is greater than the cross-section of said middle tube (14), said outer tube (16) being disposed concentrically and coaxially with said middle tube (14) and said inner tube (12); a manifold (24) securing and encapsulating each of said proximal ends of said middle and outer tubes (42,50); and a plurality of extension tubes (30,32) coupled to said manifold (24), each separately coupled to the second and third lumen (20,22) respectively,
characterized in that
said distal end (38) of said outer tube (16) is tapered and abuts said middle tube (14), and said distal end (36) of said middle tube (14) extends longitudinally outward from said distal end of said outer tube (16), wherein said middle tube (14) is tapered and abuts said inner tube (12), wherein said second lumen (20) terminates in a side opening (54) slightly upstream from said tapered distal end (36) of said middle tube (14), and said third lumen (22) terminates in a side opening (56) slightly upstream from said tapered distal end (38) of said outer tube (16), the said manifold (24) also securing and encapsulating the said proximal end (40) of the inner tube (12) wherein the inner lumen (18) is coupled to an extension tube (28) through the manifold (24).

2. Multilumen catheter (10) comprising an inner catheter tube (12) defining a first lumen (18) having a proximal end (40) and a distal end (34); a middle catheter tube (14) defining a second lumen (20) having a proximal end (42), a distal end (36) and a cross-section which is greater than the cross-section of said inner tube (12), said middle tube (14) being disposed concentrically and coaxially with said inner tube (12), and said distal end (34) of said inner tube (12) extending longitudinally outward from said distal end (36) of said middle tube (14); an outer catheter tube (16) defining a third lumen (22) and having a proximal end (50), a distal end (38) and a cross-section which is greater than the cross-section of said middle tube (14), said outer tube (16) being disposed concentrically and coaxially with said middle tube (14) and said inner tube (12); a manifold (24) securing and encapsulating each of said proximal ends of said middle and outer tubes (42,50); and a plurality of extension tubes (30,32) coupled to said manifold (24), each seperately coupled to the second and third lumen (20,22) respectively,
characterized in that
said distal end (36) of said middle tube (14) extends longitudinally outward from said distal end (38) of said outer tube (16), and said distal end (36) of said middle tube (14) and said distal end (38) of said outer tube (16) each terminates in a flexible, tapered tip (60,62) which expands under the pressure of fluid flowing in the respective second and third lumen (20,22) and which acts as a check valve to prevent fluid from entering the second and third lumens (20,22) from the respective tips (60,62), the said manifold (24) also securing and encapsulating the said proximal end (40) of the inner tube (12) wherein the inner lumen (18) is coupled to an extension tube (28) through the manifold (24).

3. Multilumen catheter (10) according to claim 2 wherein said distal end (34) of said inner tube (12) terminates in a flexible, tapered tip (58), which acts as a check valve to prevent fluid from entering the first lumen (18) from said tip (58).

4. Multilumen catheter (10) according to claim 1 or 2 wherein said extension tubes (28,20,32) are configured for connection to a fluid transfer device or monitoring means.

5. Multilumen catheter (10) according to claim 4 wherein said manifold (24) includes channels (29,37,39) for separately coupling each of said lumens (18,20,22) and respective extension tubes (28,30,32).

6. Multilumen catheter (10) according to claim 2 wherein each of said lumens (18,20,22) terminates coextensively with said respective distal end of said inner, middle and outer tubes (12,14,16).

7. Multilumen catheter (10) according to claim 1 or 2 wherein said inner, middle and outer tubes (12,14,16) are made from flexible, sterilizable materials.

8. Multilumen catheter (10) according to claim 7 wherein said inner and middle tubes (12,14) are made from a less rigid material than said outer tube (16).

9. Multilumen catheter (10) according to claim 8 wherein said inner and middle tubes (12,14) are made from polyurethane.

10. Multilumen catheter (10) according to claim 1 or 2 wherein said manifold (24) is molded.

11. Multilumen catheter (10) according to claim 1 or 2 wherein said manifold (24) is made in separate molded sections.

12. Multilumen catheter (10) according to claim 11 wherein said separate molded sections are first, second and third hubs (46,48,52); said first hub (46) defining a channel (29) which receives and secures the inner catheter tube (12) in fluid-tight communication with said respective extension tubing (28); said second hub (48) defining an annular space (31) which receives and secures the middle catheter tube (14) in fluid-tight communication with said respective extension tubing (30); and said third hub (52) defining an annular space (33) which receives and secures the outer catheter tube (16) in fluid-tight communication with said respective extension tubing (32).

13. Multilumen catheter (10) according to claim 1 or 2 wherein a manifold (24a) secures en encapsualtes a coupled portion of each extension tube, said manifold facilitating fluid communication between each of said lumens and respective extension tubing.

14. Multilumen catheter (10) according to claim 1 or 2 wherein said proximal end of said inner tube (40) extends beyond said proximal end of said middle tube (42).

15. Multilumen catheter (10) according to claim 14 wherein said proximal end of said middle tube (42) extends beyond said proximal end of said outer tube (50).

## Patentansprüche

1. Mehrfachlumen-Katheter (10) mit einer inneren Katheterröhre (12), die ein erstes Lumen (18) mit einem Proximalende (40) und einem Distalende (34) begrenzt; einer mittleren Katheterröhre (14), die ein zweites Lumen (20) mit einen Proximalende (42), einem Distalende (36) und einem Querschnitt begrenzt, der größer als der Querschnitt der inneren Röhre (12) ist, wobei die mittlere Röhre (14) konzentrisch und koaxial zur inneren Röhre (12) angeordnet ist und das Distalende (34) der inneren Röhre (12) sich in Langsrichtung auswärts vom Distalende (36) der mittleren Röhre (14) erstreckt; einer äußeren Katheterröhre (16), die ein drittes Lumen (22) mit einem Proximalende (50), einem Distalende (38) und einem Querschnitt begrenzt, der größer als der Querschnitt der mittleren Röhre (14) ist, wobei die äußere Röhre (16) konzentrisch und koaxial zur mittleren Röhre (14) und zur inneren Röhre (12) angeordnet ist; einem Sammelorgan (24), das jedes der Proximalenden der mittleren und äußeren Röhren (42,50) befestigt und einschließt; und mehreren mit dem Sammelorgan (24) verbundenen Verlängerungsröhren (30,32), die je einzeln mit dem zweiten bzw. dritten Lumen (20,22) verbunden sind, dadurch gekennzeichnet, daß das Distalende (38) der äußeren Röhre (16) sich verjüngt und an der mittleren Röhre (14) anliegt, und das Distalende (36) der mittleren Röhre (14) sich in Längsrichtung auswärts vom Distalende der äußeren Röhre (16) erstreckt, wobei die mittlere Röhre (14) sich verjüngt und an der inneren Röhre (12) anliegt, wobei das zweite Lumen (20) in einer Seitenöffnung (54) endet, die sich ein wenig stromaufwärts vom verjüngten Distalende (36) der mittleren Röhre (14) befindet, und das dritte Lumen (22) in einer Seitenöffnung (56) endet, die sich ein wenig stromaufwärts vom verjüngten Distalende (38) der äußeren Röhre (16) befindet, wobei das Sammelorgan (24) auch das Proximalende (40) der inneren Röhre (12) befestigt und einschließt und das innere Lumen (18) mit einer Verlängerungsröhre (28) durch das Sammelorgan (24) verbunden ist.

2. Mehrfachlumen-Katheter (10) mit einer inneren Katheterröhre (12), die ein erstes Lumen (18) mit einem Proximalende (40) und einem Distalende (34) begrenzt; einer mittleren Katheterröhre (14), die ein zweites Lumen (20) mit einen Proximalende (42), einem Distalende (36) und einem Querschnitt begrenzt, der größer als der Querschnitt der inneren Röhre (12) ist, wobei die mittlere Röhre (14) konzentrisch und koaxial zur inneren Röhre (12) angeordnet ist und das Distalende (34) der inneren Röhre (12) sich in Längsrichtung auswärts vom Distalende (36) der mittleren Röhre (14) erstreckt; einer äußeren Katheterröhre (16), die ein drittes Lumen (22) mit einem Proximalende (50), einem Distalende (38) und einem Querschnitt begrenzt, der größer als der Querschnitt der mittleren Röhre (14) ist, wobei die äußere Röhre (16) konzentrisch und koaxial zur mittleren Röhre (14) und zur inneren Röhre (12) angeordnet ist; einem Sammelorgan (24), das jedes der Proximalenden der mittleren und äußeren Röhren (42,50) befestigt und einschließt; und mehreren mit dem Sammelorgan (24) verbundenen Verlängerungsröhren (30,32), die je einzeln mit dem zweiten bzw. dritten Lumen (20,22) verbunden sind, dadurch gekennzeichnet, daß das Distalende (36) der mittleren Röhre (14) sich in Längsrichtung auswärts vom Distalende (38) der äußeren Röhre (16) erstreckt und das Distalende (36) der mittlere Röhre (14) und das Distalende (38) der äußeren Röhre (16) je in einer flexiblen, verjüngten Spitze (60,62) endet, die sich unter dem Druck einer im zweiten bzw. dritten Lumen (20,22) strömenden Flüssigkeit erweitert und als Absperrventil wirksam ist, um zu verhindern, daß Flüssigkeit von den jeweiligen Spitzen (60,62) in das zweite und dritte Lumen (20,22) eintritt, wobei das Sammelorgan (24) auch das Proximalende (40) der inneren Röhre (12) befestigt und einschließt und das innere Lumen (18) mit einer Verlängerungsröhre (28) durch das Sammelorgan (24) verbunden ist.

3. Mehrfachlumen-Katheter (10) nach Anspruch 2, wobei das Distalende (34) der inneren Röhre (12) in einer flexiblen, verjüngten Spitze (58) endet, die als Absperrventil wirksam ist, um zu verhindern, daß Flüssigkeit von der Spitze (58) in das erste Lumen (18) eintritt.

4. Mehrfachlumen-Katheter (10) nach Anspruch 1 oder 2, wobei die Verlängerungsröhren (28,20,32) zum Anschluß an eine Flüssigkeitszuführeinrichtung oder ein Überwachungsorgan gestaltet sind.

5. Mehrfachlumen-Katheter (10) nach Anspruch 4, wobei das Sammelorgan (24) Kanäle (29,37,39) enthält, um jedes der Lumen (18,20,22) und jeweilige Verlängerungsröhren (28,30,32) einzeln zu verbinden.

6. Mehrfachlumen-Katheter (10) nach Anspruch 2, wobei jedes der Lumen (18,20,22) koextensiv zum jeweiligen Distalende der inneren, mittleren und äußeren Röhren (12,14,16) endet.

7. Mehrfachlumen- Katheter (10) nach Anspruch 1 oder 2, wobei die inneren, mittleren und äußeren Röhren (12,14,16) aus flexiblen, sterilisierbaren Materialien hergestellt sind.

8. Mehrfachlumen-Katheter (10) nach Anspruch 7, wobei die inneren und mittleren Röhren (12,14,16) aus einem weniger starren Material hergestellt sind als die äußere Röhre (16).

9. Mehrfachlumen-Katheter (10) nach Anspruch 8, wobei die inneren und mittleren Röhren (12,14,16) aus Polyurethan hergestellt sind.

10. Mehrfachlumen- Katheter (10) nach Anspruch 1 oder 2, wobei das Sammelorgan (24) gespritzt ist.

11. Mehrfachlumen-Katheter (10) nach Anspruch 1 oder 2, wobei das Sammelorgan (24) in einzelnen gespritzten Abschnitten hergestellt ist.

12. Mehrfachlumen-Katheter (10) nach Anspruch 11, wobei die einzelnen gespritzten Abschnitte erste, zweite und dritte Buchsen (46,48,52) sind; wobei die erste Buchse (46) einen Kanal (29) begrenzt, der die innere Katheterröhre (12) in flüssigkeitsdichter Verbindung mit der jeweiligen Verlängerungsröhre (28) aufnimmt und befestigt; die zweite Buchse (48) einen Ringraum (31) begrenzt, der die mittlere Katheterröhre (14) in flüssigkeitsdichter Verbindung mit der jeweiligen Verlängerungsröhre (30) aufnimmt und befestigt; und die dritte Buchse (52) einen Ringraum (33) begrenzt, der die äußere Katheterröhre (16) in flüssigkeitsdichter Verbindung mit der jeweiligen Verlängerungsröhre (32) aufnimmt und befestigt.

13. Mehrfachlumen-Katheter (10) nach Anspruch 1 oder 2, wobei ein Sammelorgan (24a) einen verbundenen Abschnitt jeder Verlängerungsröhre befestigt und einschließt, welches Sammelorgan die Flüssigkeitsverbindung zwischen jedem der Lumen und der jeweiligen Verlängerungsröhre erleichtert.

14. Mehrfachlumen-Katheter (10) nach Anspruch 1 oder 2, wobei das Proximalende der inneren Röhre (40) sich am Proximalende der mittleren Röhre (42) vorbei erstreckt,

15. Mehrfachlumen- Katheter (10) nach Anspruch 14, wobei das Proximalende der mittleren Röhre (42) sich am Proximalende der äußeren Röhre (50) vorbei erstreckt.

## Revendications

1. Cathéter (10) à canaux multiples comprenant un tube cathéter intérieur (12) définissant un premier canal (18) ayant une extrémité proximale (40) et une extrémité distale (34) ; un tube cathéter intermédiaire (14) définissant un deuxième canal (20) ayant une extrémité proximale (42), une extrémité distale (36) et une section transversale supérieure à la section transversale dudit tube intérieur (12), ledit tube intermédiaire (14) étant disposé concentriquement et coaxialement audit tube intérieur (12), et ladite extrémité distale (34) dudit tube intérieur (12) s'étendant longitudinalement vers l'extérieur depuis ladite extrémité distale (36) dudit tube intermédiaire (14) ; un tube cathéter extérieur (16) définissant un troisième canal (22) et ayant une extrémité proximale (50), une extrémité distale (38) et une section transversale supérieure à la section transversale dudit tube intermédiaire (14), ledit tube extérieur (16) étant disposé concentriquement et coaxialement audit tube intermédiaire (14) et audit tube intérieur (12) ; un dispositif (24) de raccordement de canaux solidarisant et encapsulant chacune desdites extrémités proximales desdits tubes extérieur et intermédiaire (42, 50) ; et des tubes d'extension (30, 32) reliés audit dispositif (24) de raccordement de canaux, chacun étant relié séparément respectivement au deuxième et au troisième canal (20, 22),
caractérisé en ce que
ladite extrémité distale (38) dudit tube extérieur (16) présente un rétrécissement en contact avec ledit tube intermédiaire (14), et ladite extrémité distale (36) dudit tube intermédiaire (14) s'étend longitudinalement vers l'extérieur depuis ladite extrémité distale dudit tube extérieur (16), en ce que ledit tube intermédiaire (14) présente un rétrécissement en contact avec ledit tube intérieur (12), en ce que ledit deuxième canal (20) se termine par une ouverture latérale (54) légèrement en amont de ladite extrémité distale (36) présentant un rétrécissement dudit tube intermédiaire (14), et ledit troisième canal (22) se termine par une ouverture latérale (56) légèrement en amont de ladite extrémité distale (38) présentant un rétrécissement dudit tube extérieur (16), ledit dispositif (24) de raccordement de canaux solidarisant et encapsulant également ladite extrémité proximale (40) du tube intérieur (12) d'où le canal intérieur (18) est relié à un tube d'extension (28) à travers ledit dispositif (24) de raccordement de canaux.

2. Cathéter (10) à canaux multiples comprenant un tube cathéter intérieur (12) définissant un premier canal (18) ayant une extrémité proximale (40) et une extrémité distale (34), un tube cathéter intermédiaire (14) définissant un second canal (20) ayant une extrémité proximale (42), une extrémité distale (36) et une section transversale supérieure à la section transversale dudit tube intérieur (12), ledit tube intermédiaire (14) étant disposé concentriquement et coaxialement audit tube intérieur (12), et ladite extrémité distale (34) dudit tube intérieur (12) s'étendant longitudinalement vers l'extérieur depuis ladite extrémité distale (36) dudit tube intermédiaire (14) ; un tube cathéter extérieur (16) définissant un troisième canal (22) et ayant une extrémité proximale (50), une extrémité distale (38) et une section transversale supérieure à la section transversale dudit tube intermédiaire (14), ledit tube extérieur (16) étant disposé concentriquement et coaxialement audit tube intermédiaire (14) et audit tube intérieur (12) ; un dispositif (24) de raccordement de canaux solidarisant et encapsulant chacune des extrémités proximales desdits tubes extérieur et intermédiaire (42, 50) ; et des tubes d'extension (30, 32) reliés auxdits dispositifs (24) de raccordement de canaux, chacun étant relié séparément respectivement au deuxième et au troisième canal (20, 22),
caractérisé en ce que
ladite extrémité distale (36) dudit tube intermédiaire (14) s'étend longitudinalement vers l'extérieur depuis ladite extrémité distale (38) dudit tube extérieur (16), et ladite extrémité distale (36) dudit tube intermédiaire (14) et ladite extremité distale (38) dudit tube extérieur (16) se terminent chacune par un embout (60, 62) souple présentant un rétrécissement, qui s'expanse sous la pression du fluide s'écoulant respectivement dans le deuxième et le troisième canal (20, 22) et qui fonctionne comme une soupape de retenue pour empêcher tout fluide de pénétrer dans le deuxième et le troisième canal (20, 22) depuis les embouts respectifs (60, 62), ledit dispositif (24) de raccordement de canaux solidarisant et encapsulant également ladite extrémité proximale (40) dudit tube intérieur (12) par où ledit canal intérieur (18) est relié à un tube d'extension (28) à travers le dispositif (24) de raccordement de canaux.

3. Cathéter (10) à canaux multiples selon la revendication 2, dans lequel ladite extrémité distale (34) dudit tube intérieur (12) se termine par un embout (58) souple présentant un rétrécissement, qui fonctionne comme une soupape de retenue pour empêcher tout fluide de pénétrer dans le premier canal (18) depuis ledit embout (58).

4. Cathéter (10) à canaux multiples selon la revendication 1 ou 2, dans lequel lesdits tubes d'extension (28, 30, 32) sont adaptés à connecter un dispositif de transfert de fluide ou des moyens de contrôle.

5. Cathéter (10) à canaux multiples selon la revendication 4, dans lequel ledit dispositif (24) de raccordement de canaux comporte des conduits (29, 37, 39) pour relier séparément chacun desdits canaux (18, 20, 22) et lesdits tubes d'extension respectifs (28, 30, 32).

6. Cathéter (10) à canaux multiples selon la revendication 2, dans lequel chacun desdits canaux (18, 20, 22) se termine co-extensivement par lesdites extrémités distales respectives desdits tubes intérieur, intermédiaire et extérieur (12, 14, 16).

7. Cathéter (10) à canaux multiples selon la revendication 1 ou 2, dans lequel lesdits tubes intérieur, intermédiaire et extérieur (12, 14, 16) sont constitués en des matériaux stérilisables souples.

8. Cathéter (10) à canaux multiples selon la revendication 7, dans lequel lesdits tubes intérieur et intermédiaire (12, 14) sont constitués en un matériau moins rigide que ledit tube extérieur (16).

9. Cathéter (10) à canaux multiples selon la revendication 8, dans lequel lesdits tubes intérieur et intermédiaire (12, 14) sont constitués en polyuréthane.

10. Cathéter (10) à canaux multiples selon la revendication 1 ou 2, dans lequel ledit dispositif (24) de raccordement de canaux est moulé.

11. Cathéter (10) à canaux multiples selon la revendication 1 ou 2, dans lequel ledit dispositif (24) de raccordement de canaux est constitué de parties moulées distinctes.

12. Cathéter (10) à canaux multiples selon la revendication 11, dans lequel lesdites parties moulées distinctes sont des premier, deuxième et troisième bulbes (46, 48, 52) ; ledit premier bulbe (46) définissant un conduit (29) qui reçoit et solidarise le tube cathéter intérieur (12) en communication étanche aux fluides avec ladite tubulure d'extension respective (28) ; ledit second bulbe (48) définissant un espace annulaire (31) qui reçoit et solidarise le tube cathéter intermédiaire (14) en communication étanche aux fluides avec ladite tubulure d'extension respective (30) ; et ledit troisième bulbe (52) définissant un espace annulaire (33) qui reçoit et solidarise le tube cathéter extérieur (16) en communication étanche aux fluides avec ladite tubulure d'extension respective (32).

13. Cathéter (10) à canaux multiples selon la revendication 1 ou 2, dans lequel un dispositif (24a) de raccordement de canaux solidarise et encapsule une partie de liaison de chaque tube d'extension, ledit dispositif de raccordement de canaux facilitant la communication de fluide entre chacun desdits canaux et la tubulure d'extension respective.

14. Cathéter (10) à canaux multiples selon la revendication 1 ou 2, dans lequel ladite extrémité proximale dudit tube intérieur (40) s'étend au-delà de ladite extrémité proximale dudit tube intermédiaire (42).

15. Cathéter (10) à canaux multiples selon la revendication (14), dans lequel ladite extrémité proximale dudit tube intermédiaire (42) s'étend au-delà de ladite extrémité proximale dudit tube extérieur (50).
